# EUROPEAN PATENT SPECIFICATION

(11) **EP 3 097 949 B1**
(45) Date of publication and mention of the grant of the patent: **29.12.2021**
(21) Application number: 16169872.5
(22) Date of filing: 17.05.2016
(51) Int. Cl.: A61N 1/39, A61B 50/00, A45C 5/06, A61B 50/31, A45C 11/00

(54) **CASE FOR MEDICAL EQUIPMENT**
GEHÄUSE FÜR MEDIZINISCHE AUSRÜSTUNG
BOÎTIER POUR ÉQUIPEMENT MÉDICAL

(30) Priority: 29.05.2015 JP 2015109446
(43) Date of publication of application: 30.11.2016
(73) Proprietor: Nihon Kohden Corporation, Shinjuku-ku Tokyo (JP)
(72) Inventor: Sato, Masashi, Tokyo (JP); Uchiyama, Yutaka, Tokyo (JP)
(74) Representative: Hoffmann Eitle

(56) References cited:
- WO-A1-2009/061663
- WO-A1-2016/081815
- US-A1- 2011 040 268
- US-A1- 2011 154 889
- US-B1- 6 422 669

## Description

### Field of Invention

The presently disclosed subject matter relates to a case for a medical equipment.

### Background Art

Ambulances and fire engines are generally equipped with a defibrillator. A defibrillator has a defibrillator main unit, electrode pads, cables for various parameters, etc. A carrying case for a defibrillator is widely used to carry these components together.

JP2013-543755A and JP2013-542034A as well as WO2009/061663 and US6422669 disclose examples of a carrying case for a defibrillator, the carrying case being made of a rigid material and configured to allow easy handling and deployment of contents.

When using a defibrillator encased in the carrying case described above, a user (1) takes the defibrillator out from the carrying case, (2) keeps the defibrillator inside the case and places the case to be in a standing state (a state in which a display is substantially perpendicular to the placement surface), or (3) keeps the defibrillator inside the case and places the case to be in a laid down state (a state in which the display is substantially parallel to the placement surface). That is, when using the defibrillator on a road or the like, the defibrillator is used in a state in which the display is substantially perpendicular to the road surface or in a state in which the display is substantially parallel to the road surface. When using the defibrillator with the display being substantially perpendicular to the placement surface, a user has to peer into the display screen from a low point. When using the defibrillator with the display being substantially parallel to the placement surface, a user also has peer into the display screen and in addition, it may be difficult to see the display screen due to incidence of light.

The problems discussed above are not limited to defibrillators, and commonly occur also with patient monitors for displaying vital signs and the like. That is, these problems are common to medical equipment having a display.

### Summary of Invention

Illustrative aspects of the present invention provide a medical equipment case which can make a display of a medical equipment easy to see during use of the medical equipment.

### Brief Description of the Drawings

Fig. 1 is a front view of a defibrillator according to an embodiment of the present invention;
Fig. 2 is a front view of a medical equipment case according to an embodiment of the present invention, with the defibrillator being provided inside the medical equipment case;
Fig. 3 is a side view of the medical equipment case in which the defibrillator is provided;
Fig. 4 is another side view of the medical equipment case in which the defibrillator is provided;
Fig. 5 is a rear view of the medical equipment case in which the defibrillator is provided;
Fig. 6 is a bottom view of the medical equipment case in which the defibrillator is provided;
Fig. 7 is a top view of the medical equipment case in which the defibrillator is provided;
Fig. 8 is a rear view of the medical equipment case, illustrating an operation for opening a support member when using the defibrillator;
Fig. 9 is a side view of the medical equipment case with the support member being opened;
Fig. 10 is a rear view of the medical equipment case with the support member being opened;
Fig. 11 is a side view of the medical equipment case in its usage state with the support member being opened;
Fig. 12 is another side view of the medical equipment case, illustrating a movement of the support member after using the defibrillator;
Fig. 13 is a right side view of a medical equipment case according to another embodiment of the present invention;
Fig. 14 is a right side view of a right side view of a medical equipment case according to another embodiment of the present invention;
Fig. 15 is a rear view of the medical equipment case of Fig. 14;
Fig. 16 is a rear view of a right side view of a medical equipment case according to another embodiment of the present invention; and
Fig. 17 is a left side view of the medical equipment case of Fig. 16.

### Embodiments of the Invention

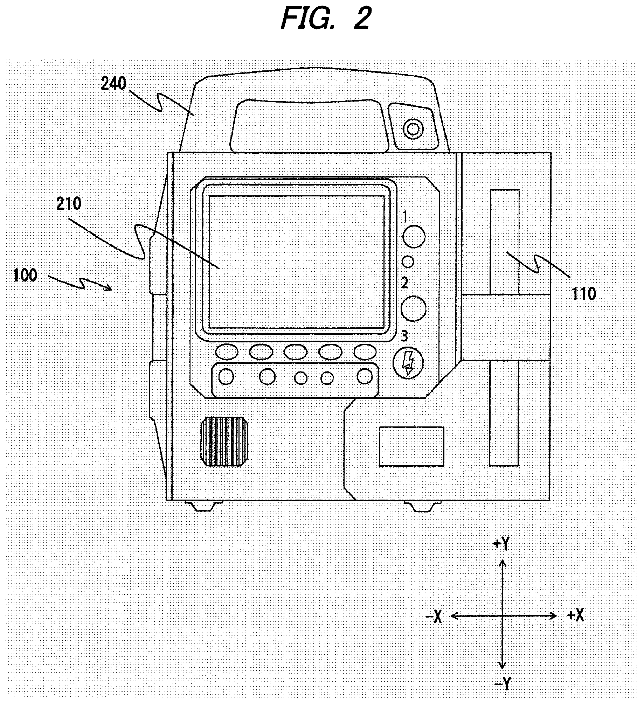

Hereinafter, embodiments of the present invention will be described with reference to the drawings. A medical equipment case 100 (which will be described later with reference to Fig. 2 and the like) of the embodiment is a housing case which can be used for various types of medical equipment such as a defibrillator and a patient monitor. In the following, an example in which the medical equipment case 100 is used as a case for a defibrillator 200 will be described.

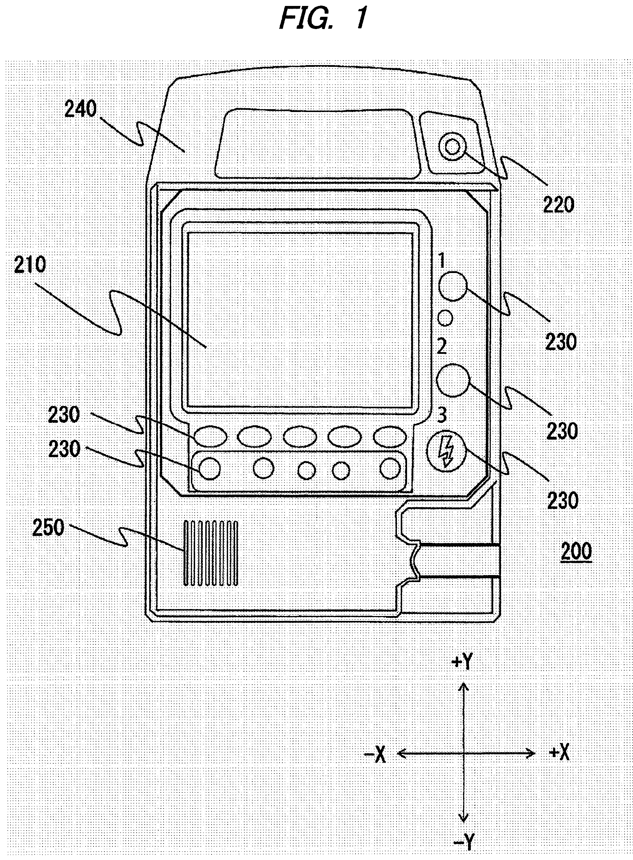

Firstly, the configuration of the defibrillator 200 which is to be housed in the medical equipment case 100 will be described with reference to Fig. 1. Fig. 1 is a front view showing the appearance of the defibrillator 200 as seen from the front side. The defibrillator 200 has a display 210, an indicator 220, an input interface 230 (e.g., buttons), a handle 240, and a speaker 250 on a housing. The defibrillator 200 adequately includes various circuits, a central processing unit (CPU), a primary storage device, a secondary storage device, and the like in the housing.

As illustrated, the display 210 is disposed in the front of the defibrillator 200. During use of the defibrillator 200, various setting screens, biological waveform information (e.g., electrocardiogram waveforms), information of measurement values (e.g., measurement values of the pulse rate and the SpO₂), and the like are displayed on the display 210. In a state in which the display 210 of the defibrillator 200 is placed in front as illustrated in Fig. 1, the lateral direction is defined as X-axis direction, the vertical direction is defined as Y-axis direction, and the depth direction (not shown) is defined as Z-axis direction.

A configuration of the medical equipment case 100 housing the defibrillator 200 will now be described with reference to Figs. 2 to 7. Figs. 2 to 7 illustrate a state in which the lower surface of the defibrillator 200 is placed on the placement surface such that the bottom surface of the medical equipment case 100 contacts the placement surface. Fig. 2 is a front view of the medical equipment case 100 in which the defibrillator 200 is provided.

In the example shown in Fig. 2, the medical equipment case 100 is configured to cover the entire surface of the housing of the defibrillator 200 other than the display 210 and the handle 240. The medical equipment case 100 may be configured in a different manner in so far as a portion of the housing of the defibrillator 200 is covered. The medical equipment case 100 houses the defibrillator 200, relevant cables and pads, etc. A housing portion 110 houses cables for various sensors, etc. The medical equipment case 100 may be made of any kind of material which exhibits both the flexibility and the performance of protecting the housing, such as polyester.

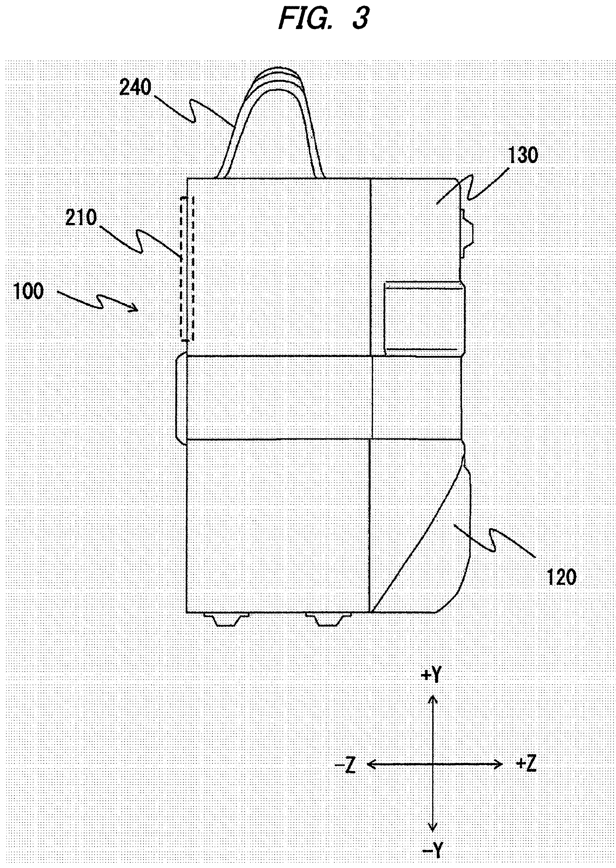

Fig. 3 is a side view of the medical equipment case 100 in which the defibrillator 200 is provided (a view of the medical equipment case 100 as seen in the +X direction). In the following views, a place which is enclosed by a broken line, and which is denoted by a reference numeral means that, although not illustrated, members are disposed at respective positions. In Fig. 3, for example, the display 210 exists in the place which is enclosed by the broken line (see Fig. 2). Although, in the example of Fig. 3, the configuration in which the display 210 is approximately perpendicular to the placement surface is shown, the angle formed by the display 210 and the placement surface may be from 70 degrees to 90 degrees. That is, the display 210 may be arranged in a slightly inclined orientation in a state in which the defibrillator 200 is vertically placed.

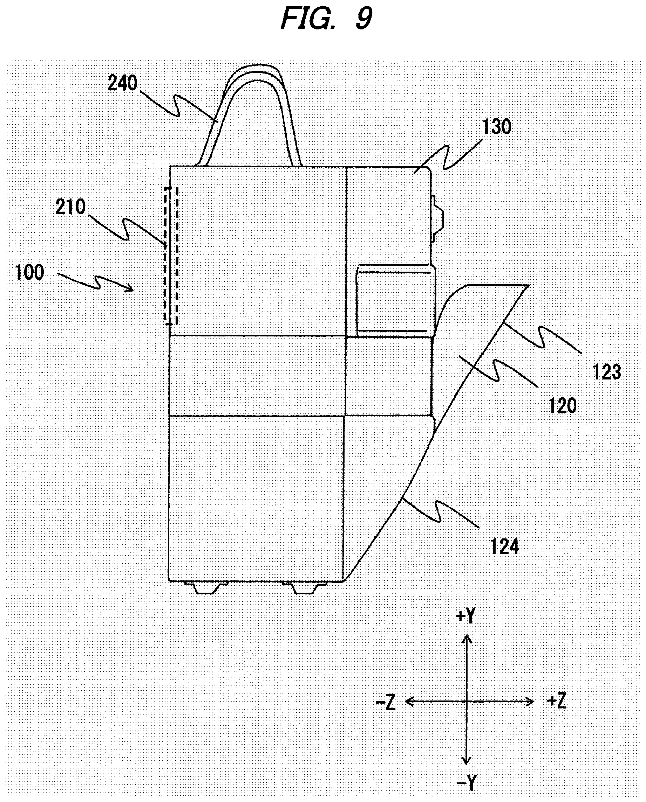

A housing portion 130 is disposed on the side of the +Z direction, and houses various cables and sensors. The medical equipment case 100 has a support member 120 at a lower portion (-Y side) on a side (+Z side) opposite to the side on which the display 210 is disposed. The support member 120 forms a portion of the medical equipment case 100. The support member 120 is configured such that a portion of the support member is connected to the case body, and the other part is attachable to and detachable from the case body as described later with reference to Figs. 9 to 11. During use of the defibrillator 200, the support member 120 is set in the state where a part is detached from the case body, thereby supporting the defibrillator 200. The supporting of the housing by the support member 120 will be described in detail later with reference to Figs. 9 to 11.

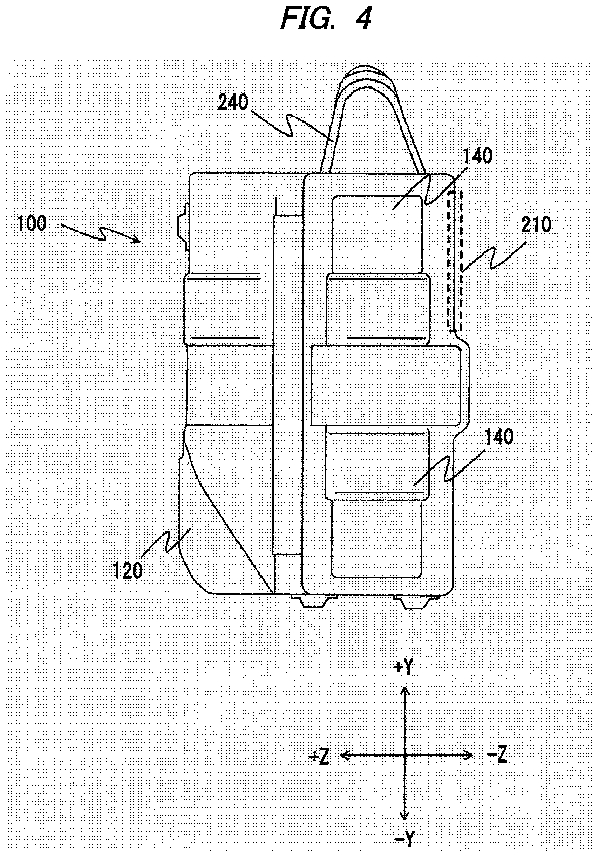

Fig. 4 is a side view of the medical equipment case 100 in which the defibrillator 200 is provided (a view of the medical equipment case 100 as seen in the -X direction). A housing portion 140 in which a battery for the defibrillator 200 is housed is disposed on the side surface of the medical equipment case 100. Similarly with Fig. 3, the support member 120 is disposed at the lower portion (the -Y side, in other words, the side in the direction of the gravitational force) on a side (+Z side) opposite to the side on which the display 210 is disposed. As shown in Figs. 3 and 4, in a state in which the support member 120 is fixed to the case body (in a state in which the entire support member 120 is connected to the case body, in other words, the contact surface (attachment and detachment surface) of the support member 120 is fixed to (in contact with) the case body), the surface opposite to the side on which the display 210 is disposed has a substantially planar shape. Here, "substantially planar shape" means that, when the bottom surface of the medical equipment case 100 is placed so as to be in contact with the placement surface, there is no protruding portion that may hit the wall or the like (a non-bulky state) although a small number of projections such as rubber feet exist.

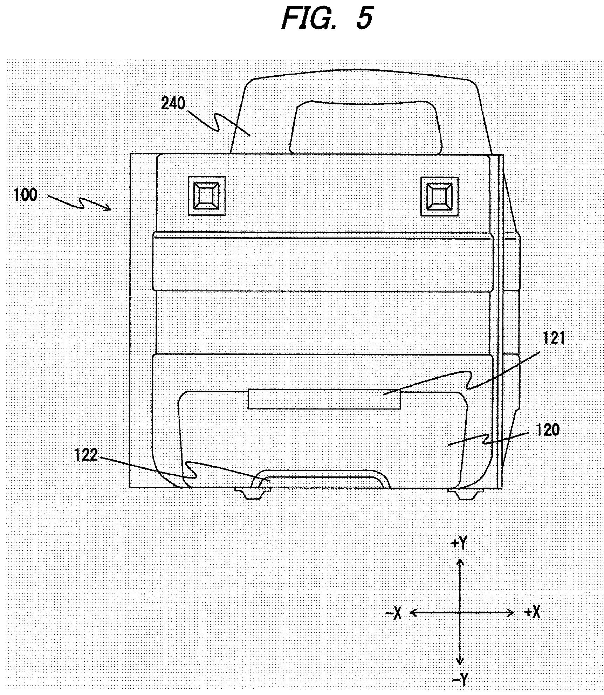

Fig. 5 is a back view of the medical equipment case 100 in which the defibrillator 200 is provided (a view of the medical equipment case 100 as seen in the +Z direction). As shown in Figs. 3 and 4, the support member 120 is disposed at the lower portion (on the -Y side) of the medical equipment case 100. Although not denoted by a reference numeral, rubber feet for absorbing shock occurring in placement may be provided on the back and bottom surfaces of the medical equipment case 100.

The support member 120 has a connecting portion 121 which is used for connecting the case body of the medical equipment case 100 to the support member 120. For example, the connecting portion 121 is connected (preferably, sawn) to the case body, and connected (preferably, sawn) also to a portion of the support member 120. When a portion of the support member 120 is to be detached, the connecting portion 121 functions as a fulcrum. The attachment and detachment of the support member 120 will be described later with reference to Figs. 9 to 12.

A cutout portion 122 configured as a space into which the fingers (or the palm) of the user are to be inserted is provided at the lower portion (at the -Y side) of the support member 120. When the defibrillator 200 is to be used, the user inserts the fingers or the hand into the cutout portion 122, and then pulls the support member 120 in an obliquely upward direction (the +Z direction (toward the front in Fig. 5) and the +Y direction). This produces a state in which only the connecting portion 121 of the support member 120 is connected to the case body, and the other portion is detached from the case body. This attachment and detachment will be further described later with reference to Figs. 9 to 12.

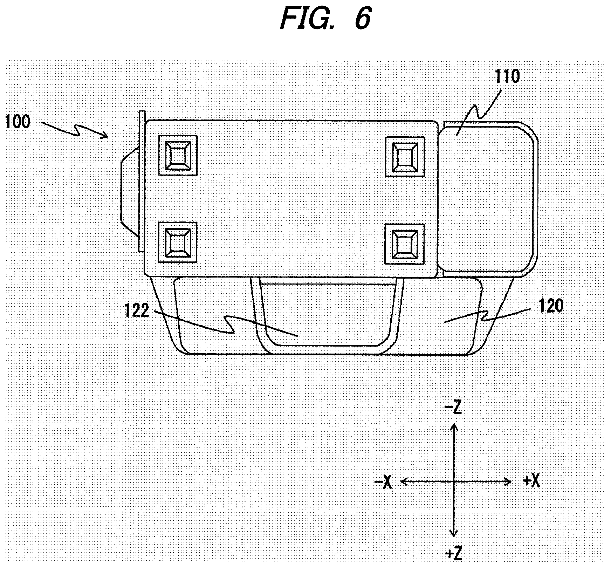

Fig. 6 is a bottom view of the medical equipment case 100 in which the defibrillator 200 is provided (a view of the medical equipment case 100 as seen in the -Y direction (i.e., the placement surface direction)). As described above, the support member 120 is disposed on the side of the placement surface. Moreover, the cutout portion 122 that is a space (cavity) into which the user is to insert the fingers or the palm is disposed in the support member 120.

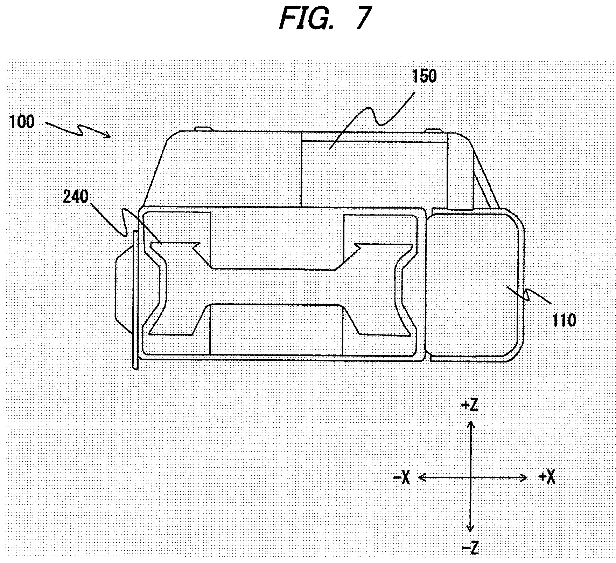

Fig. 7 is a top view of the medical equipment case 100 in which the defibrillator 200 is provided (a view of the medical equipment case 100 as seen in the +Y direction). As shown also in Fig. 2 and the like, the handle 240 is exposed from the medical equipment case 100. A housing portion 150 into which various cables and the like are inserted from the upper side (the +Y side) is disposed in the medical equipment case 100.

In the above, the basic configuration of the medical equipment case 100 has been described with reference to the six-directional views (Figs. 2 to 7). In the following, the form of the medical equipment case 100 during use of the defibrillator 200 will be described. During non-use of the defibrillator 200 (e.g., when mounted in a fire engine or the like), the defibrillator is mounted such that the support member 120 is closely attached to the case body of the medical equipment case 100 (Figs. 3 and 4).

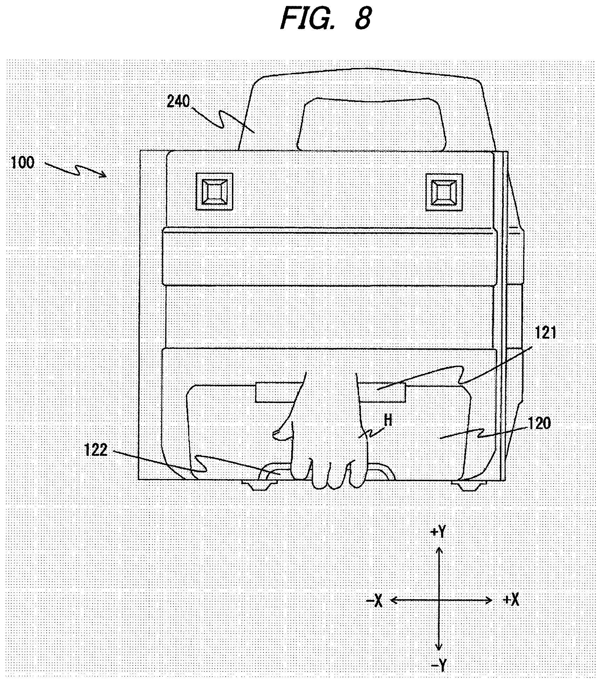

Fig. 8 is a view illustrating an operation of setting the support member 120 to an opened state when the defibrillator 200 is to be used. Fig. 8 shows an operation as seen from the back side of the medical equipment case 100. As illustrated, the user (e.g., an emergency medical technician) inserts the fingers of the hand H into the cutout portion 122. Namely, the user inserts the hand H into the cutout portion 122 in order to grasp (pinch, grip, hold, or hook by the fingers) the support member 120. Then, the user lifts the support member 120 such that the hand H is lifted in the +Z direction (the forward direction in Fig. 8) and the +Y direction. This lifting operation causes the support member 120 to be in a state in which the connecting portion 121 remains to be connected to the case body and the other portion of the support member 120 is detached therefrom.

Fig. 9 is a side view illustrating a state in which a portion of the support member 120 is detached from the case body of the medical equipment case 100 (a view of the medical equipment case 100 as seen in the +X direction). As illustrated, the support member 120 forms a convex portion that is convex in the direction (+Z direction) away from the display 210. When viewing from the lateral side (in the +X or -X direction), namely, a state where the support member 120 having a substantially triangular shape is projected in the +Z direction from the surface parallel to the display 210 is obtained. Furthermore, the contact surface 123 of the support member 120, and a contact surface 124 of the case body form a substantially planar shape (the linear shape in Fig. 9).

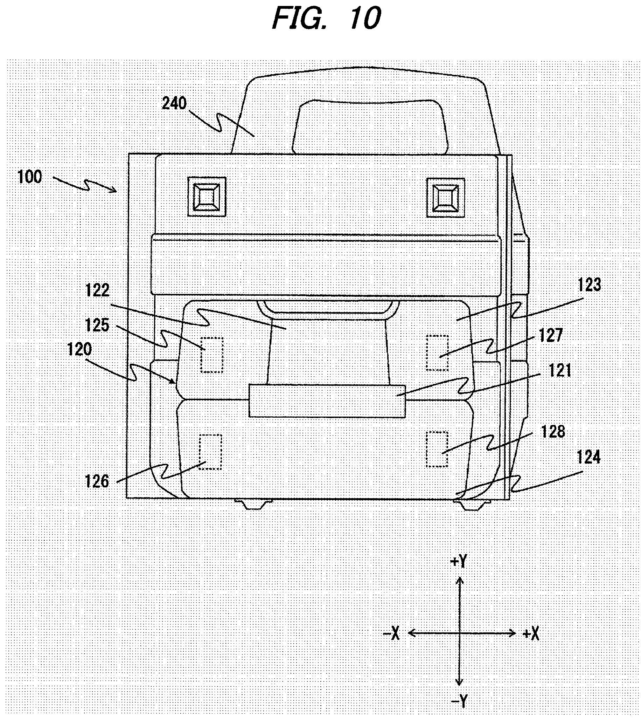

Fig. 10 illustrates a configuration in which a portion of the support member 120 is detached, as seen from the back side. As illustrated, the support member 120 is connected to the case body through the connecting portion 121, and the other portion (the portion other than the connecting portion 121) is separated from the case body such that the portion everts toward the upper side (in the +Y direction). As shown in Fig. 9, the contact surface 124 on the side of the case body of the medical equipment case 100, and the contact surface 123 on the side of the support member 120 are configured.

Preferably, a fastener 125 and a fastener 127 are disposed on the contact surface 123. Similarly, it is preferable that a fastener 126 and a fastener 128 are disposed on the contact surface 124. The fastener 125 and the fastener 126 are provided at locations that correspond to each other (i.e., the locations where they are opposed to each other when the contact surface 123 and the contact surface 124 are in contact with each other). Similarly, the fastener 127 and the fastener 128 are provided at locations that correspond to each other (i.e., the locations where they are opposed to each other when the contact surface 123 and the contact surface 124 are in contact with each other).

The fasteners 125 to 128 are configured to firmly attached the support member 120 to the case body of the medical equipment case 100, and may be, for example, magnets. Since the fastener 125 and the fastener 126 are attracted to each other by their magnetic forces, the support member 120 is firmly attached to the case body when the defibrillator 200 is vertically placed (when placed such that the bottom surface is in contact with the placement surface as shown in Figs. 2 to 7). Similarly, since the fastener 127 and the fastener 128 are attracted to each other by their magnetic forces, the support member 120 is firmly attached to the case body when the medical equipment case 100 is vertically placed. The example of Fig. 10 has two sets of fasteners. However, the number of sets of fasteners is not limited to two, and at least one set of fasteners may be provided on the contact surfaces.

The fasteners 125 to 128 may be configured as other type of fasteners, not necessarily magnetic fasteners, in so far as they can firmly attach the support member 120 to the case body of the medical equipment case 100. For example, the fasteners may be hook and loop fasteners.

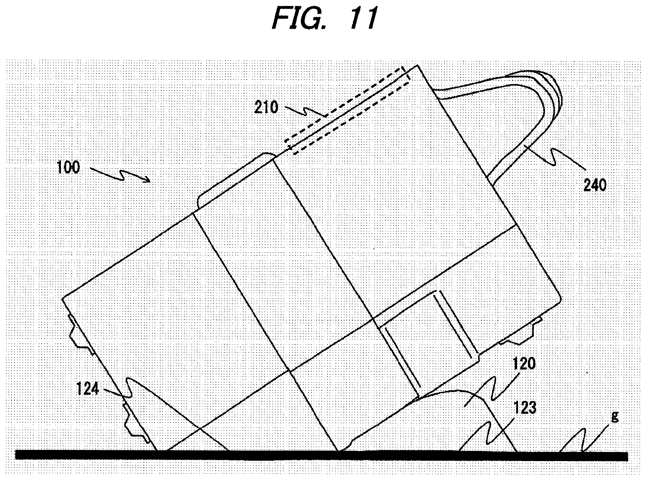

Next, a use mode in which the defibrillator 200 is used will be described with reference to Fig. 11. Before use of the defibrillator 200, the user sets a state where the support member 120 is lifted (the state of Fig. 9), and, while maintaining this state, then places the medical equipment case 100 so that the contact surface 123 and the contact surface 124 are contacted with the placement surface g (Fig. 11). Here, the placement surface g means mainly the ground surface or the like, and is a plane on which the defibrillator 200 is placed when emergency medical activity is to be performed.

When the medical equipment case 100 is placed so that the contact surface 123 and the contact surface 124 are contacted with the placement surface g, the support member 120 supports the load of the defibrillator 200. Since the support member 120 supports the load of the defibrillator 200, the display 210 is inclined with respect to the placement surface g. In other words, the support member 120 supports the load of the defibrillator 200 so that the display 210 is inclined with respect to the placement surface g.

Here, the support member 120 supports the load of the defibrillator 200, and therefore the support member 120 preferably has a rigidity by which the shape of the support member 120 is prevented from being deformed by the load. In order to prevent the case body of the medical equipment case 100, and the housing of the defibrillator 200 from being damaged, the support member 120 is preferably configured by a cushion member (a member having elasticity) having also the cushion property. Therefore, the support member 120 is preferably configured by plastic or the like having the rigidity.

Since the display 210 is inclined with respect to the placement surface g, the user (mainly an emergency medical technician who uses the defibrillator 200) can easily refer various kinds of information displayed on the display 210.

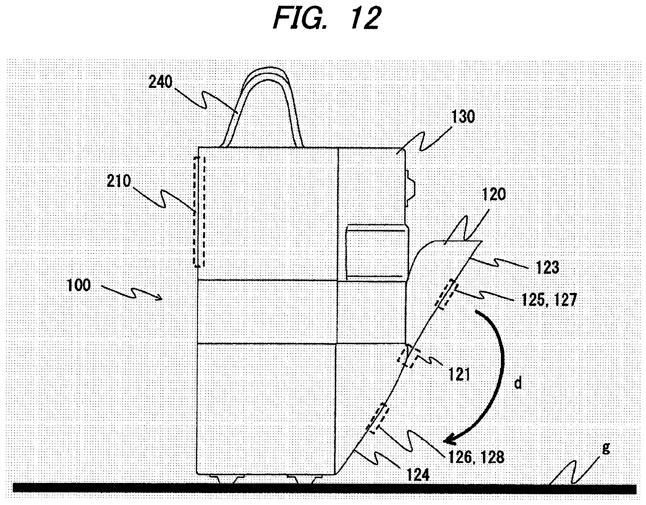

Fig. 12 is a view illustrating the movement of the support member 120 after use of the defibrillator 200. The user grasps the handle 240 and sets the medical equipment case 100 such that the bottom surface of the medical equipment case 100 is in contact with the placement surface g (the state shown in Fig. 12).

In this state, the gravitational force in the direction of the placement surface g is applied to the support member 120. Therefore, the support member 120 is moved in the direction d while using the connecting portion 121 as a fulcrum. With the magnetic fasteners 125 to 128, the fastener 125 and the fastener 126 are attracted to each other, and the fastener 127 and the fastener 128 are attracted to each other, whereby the contact surface 123 and the contact surface 124 are attracted to each other and are firmly attached to each other.

Also in a case in which the fasteners 125 to 128 are hook and loop fasteners, the support member 120 is moved in the direction d by the gravity as shown in Fig. 12. When the contact surface 123 and the contact surface 124 are contacted to each other by the gravity, the fasteners 125 to 128, i.e. the hook and loop fasteners, are firmly attached to each other.

By placing the defibrillator 200 in a vertical manner such that the bottom surface of the medical equipment case 100 is in contact with the placement surface g, the support member 120 is automatically attached to the case body, even without manually operating the support member 120 (no force applied by hand). After use of the defibrillator 200, namely, the user can cause the support member 120 to be fixed to the case body simply by slightly lifting the handle 240.

The medical equipment case 100 having the configuration described above may be modified in various manner.

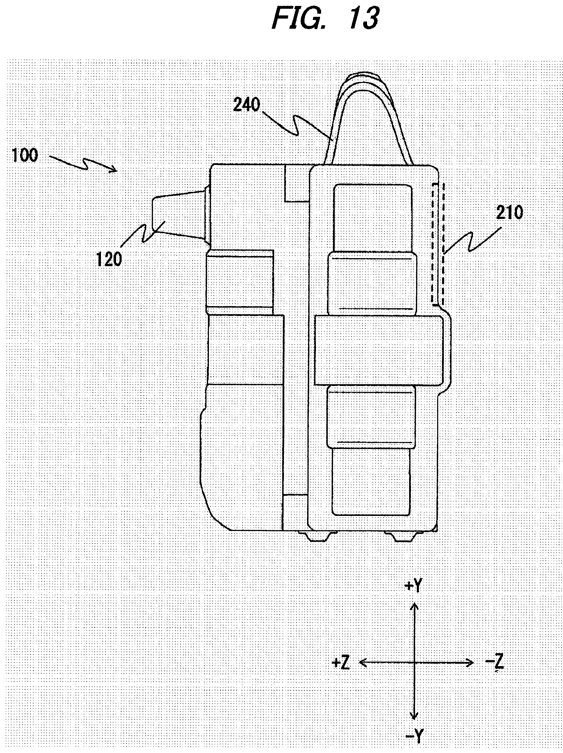

For example, while the support member 120 is attachable to and detachable from the case body to support the display 210 in an inclined orientation, the support member 120 may be provided in a different form. Fig. 13 is a side view of a modified example of the support member 120 (a view of the medical equipment case 100 as seen in the -X direction).

In the configuration shown in Fig. 13, when the defibrillator 200 is vertically placed such that the bottom surface of the medical equipment case 100 is in contact with the placement surface g, the support member 120 is convex in the +Z direction (the direction away from the display 210) (in other words, the support member 120 forms a convex portion). Also with this configuration, during use of the defibrillator 200, the support member 120 supports the load of the defibrillator 200, whereby the display 210 is caused to be inclined with respect to the placement surface. Therefore, the user can easily refer information displayed on the display 210 during use of the defibrillator 200. Also in the configuration, the support member 120 preferably has both the rigidity and the cushion property.

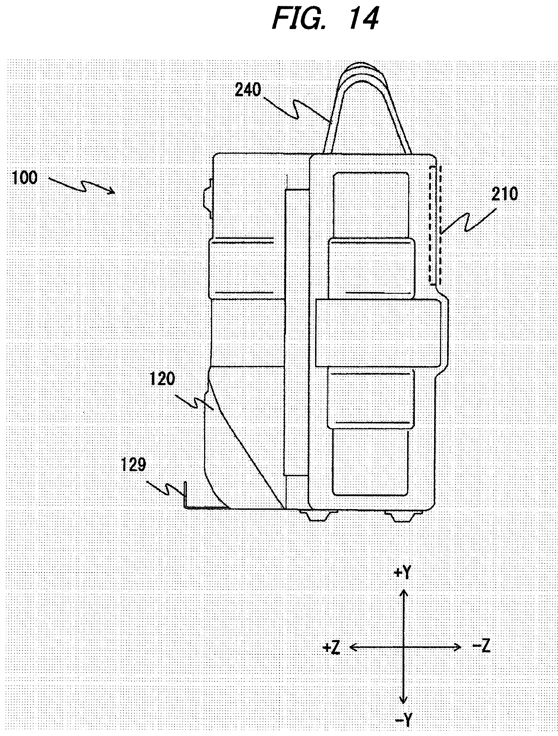

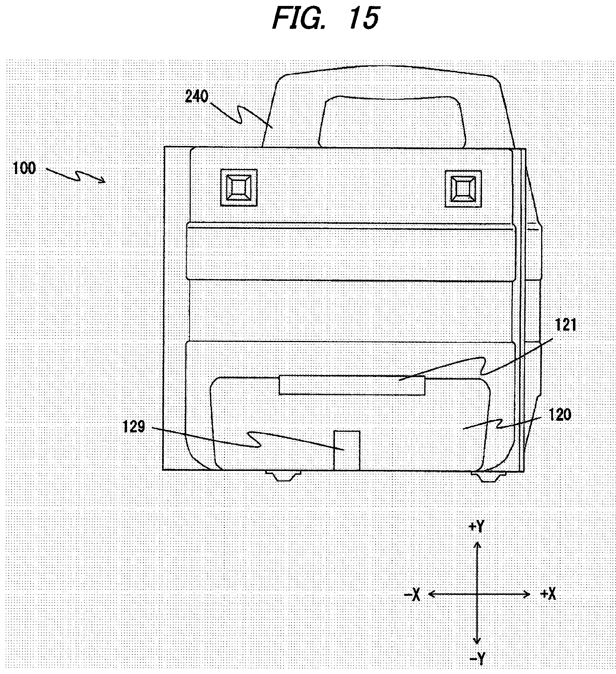

The cutout portion 122 is an example of a grasping portion adapted to be grasped by the user in order to open the support member 120 (to pull a portion of the support member 120 apart from the case body as shown in Fig. 9). The grasping portion may be configured in a different manner. A modified example of the grasping portion will be described with reference to Figs. 14 and 15.

Fig. 14 is a side view of the modified example having a pulling member 129 in place of the cutout portion 122 (a view of the medical equipment case 100 as seen in the -X direction). The pulling member 129 is fixed to the support member 120 (for example, by partially being sewn to the support member). For example, the pulling member 129 may be made from cloth having a strength which can withstand pulling applied by the user.

The user grasps the pulling member 129 (holds by strongly pinching the member), and pulls the member in an obliquely upward direction (the +Z direction and the +Y direction). This produces a state where the support member 120 is separated from the case body while using the connecting portion 121 as a fulcrum. That is, the support member 120 is in the state shown in Fig. 9. After the support member 120 is in the state where the member is separated from the case body, the user places the medical equipment case 100 so that the contact surface 123 and the contact surface 124 are contacted with the placement surface (namely, the defibrillator 200 is placed in the state shown in Fig. 11).

Fig. 15 is a back view of the configuration having the pulling member 129. As illustrated, a portion of the pulling member 129 is fixed to (preferably, sewn to) the support member 120. Preferably, the pulling member 129 is disposed in a portion of the support member 120 which is in a substantially middle in the X-axis direction, as shown in Fig. 15. Since the pulling member 129 is disposed in a substantially middle portion, the support member 120 can be surely pulled up even when a weak force is applied.

Also with the pulling member 129 disposed as the configuration for separating the support member 120 from the case body as shown in Figs. 14 and 15, the user can easily lift the support member 120. As a further modification of the pulling member 129, a configuration in which the member is formed by a rigid hook or the like in place of a flexible material such as cloth may be employed.

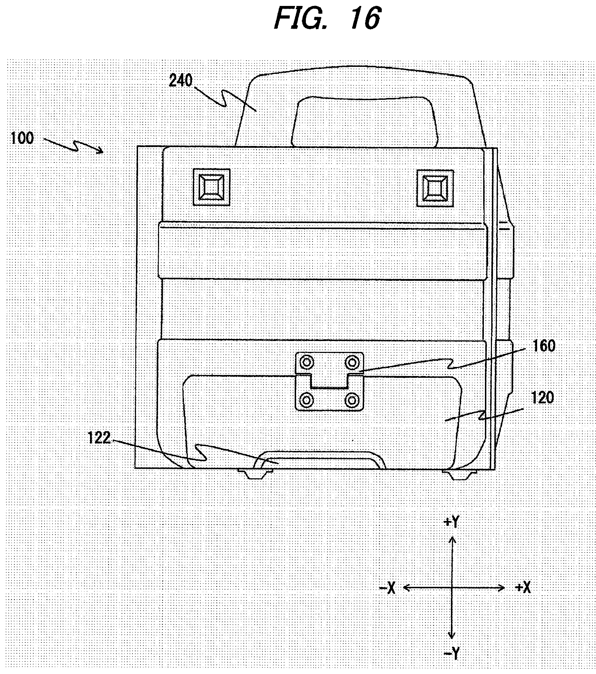

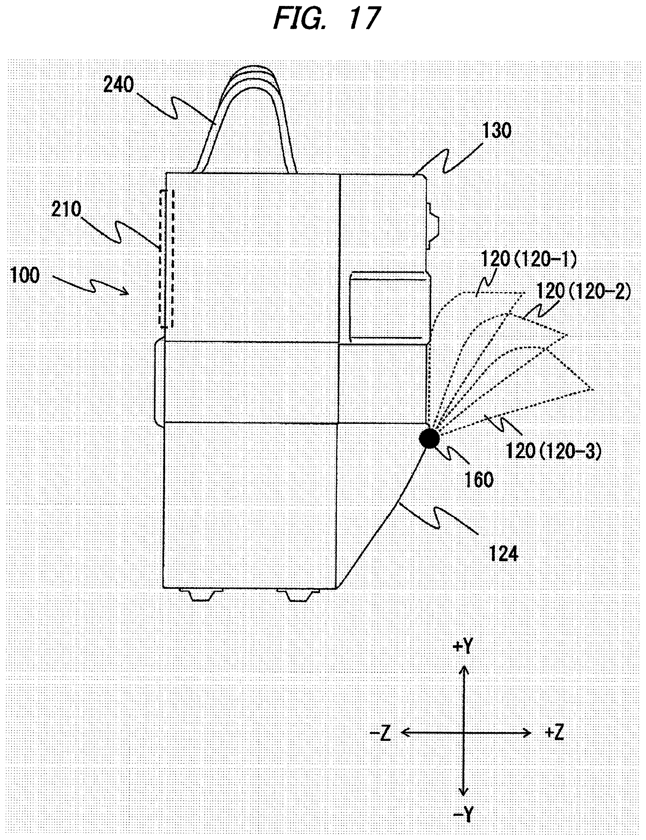

Furthermore, a configuration in which the angle at which the support member 120 supports the display 210 is variable may be employed. The configuration will be described with reference to Figs. 16 and 17. Fig. 16 is a back view of the medical equipment case 100 of the modification (a view of the medical equipment case 100 as seen in the +Z direction).

The modification (Fig. 16) has an angle adjusting mechanism 160 as a variation of the connecting portion 121. For example, the angle adjusting mechanism 160 is a torque hinge, and adjusts the angle (connection angle) at which the support member 120 is connected to the body of the medical equipment case 100. Namely, the user adjusts the degree of the force which is applied to the cutout portion 122 to grasp and lift the portion, thereby adjusting the angle at which the support member 120 is fixed to the case body. In other words, the angle adjusting mechanism 160 holds the connection angle of the support member 120 with respect to the case body.

When providing the angle adjusting mechanism 160 using a torque hinge, the torque hinge may be of the free-stop type in which the angle can be arbitrarily adjusted, or of a type in which adjustable angles are limited to several ones. The angle adjusting mechanism 160 may be configured by a mechanism other than a torque hinge as far as the mechanism can fix the support member 120 and the case body, and adjust the connection angle of the support member 120.

Fig. 17 is a side view illustrating a usage state in the example having the angle adjusting mechanism 160 (a view of the medical equipment case 100 as seen in the +X direction). In Fig. 17, in order to clarify the description, the support member 120 is indicated by the broken lines, and the angle adjusting mechanism 160 is conceptually indicated by the black circle. The support member 120 is fixed at an arbitrary angle while using the angle adjusting mechanism 160 as a fulcrum. In Fig. 17, the support member 120 can be fixed at each of angles indicated by support members 120-1 to 120-3, respectively. The user grasps the cutout portion 122, and then adjusts the angle of the support member 120, whereby the angle of the display 210 in the placement state can be arbitrarily changed.

The configuration (Figs. 16 and 17) having the angle adjusting mechanism 160 allows the user to arbitrarily change the angle of the display 210 in the placement state. Therefore, the user can adequately set the angle of the display 210 in accordance with the user's taste or the use environment.

Advantages of the medical equipment case 100 of the embodiment will be described. As described above, the medical equipment case 100 includes the support member 120 to support the load of the defibrillator 200 from a side (+Z side) opposite to a side on which the display 210 of the defibrillator 200 is disposed. In a state in which the support member 120 supports the load of the defibrillator 200, the display 210 of the defibrillator 200 is inclined (not vertical) with respect to the placement surface. The inclined orientation of the display 210 with respect to the placement surface allows a user to easily check various information displayed on the display 210. Further, the inclined orientation of the display 210 with respect to the placement surface limits light, such as sunlight, from being incident on the display 210, which reduces the difficulty in seeing the display 210 due to light reflected by the display 210.

During the vertical placement (when placed such that the bottom surface of the case is in contact with the placement surface), the support member 120 is in close contact with the case body (see, e.g., Figs. 3 and 4). According to the configuration, the depth (the Z-axis dimension) of the medical equipment case 100 is not increased. Therefore, the width is not increased during the vertical placement, and the medical equipment case 100 and the defibrillator 200 can be stored in a compact manner in an ambulance or a storage location.

In a state in which the support member 120 is closely contacted with the case body, the surface on a side opposite to the side on which the display 210 is disposed has a substantially planar shape (Figs. 3 and 4). During the vertical placement, therefore, the medical equipment case 100 is formed into a compact shape (a shape in which the width is small).

The fasteners 125, 127 and 126, 128 are disposed on the contact surface 123 of the support member 120, and the contact surface 124 of the case body of the medical equipment case 100, respectively. According to the configuration, during vertical placement of the defibrillator 200, the support member 120 can be surely fixed to the case body (Figs. 3, 4, 10, and 12). When the fasteners 125 to 128 are configured by magnets, it is possible to realize firm fixation due to magnetic forces.

Moreover, the grasping portion which is to be grasped by the user when the support member 120 is to be lifted is disposed. The term "grasping portion" is a concept including the cutout portion 122 which is configured by, for example, hollowing out the support member 120 (Figs. 5, 6, and 8), and the pulling member 129 (Figs. 14 and 15). When the user grasps the cutout portion 122 or the pulling member 129 and then lifts it, the support member 120 can be easily set to the opened state (Figs. 10 and 11).

The contact surface 123 of the support member 120, and the contact surface 124 of the case body of the medical equipment case 100 are configured into a substantially planar shape (Fig. 12). In other words, in Fig. 12, the contact surface 123 and the contact surface 124 form a substantially straight line. According to the configuration, the area which is in contact with the placement surface g during use of the defibrillator 200 is increased (Fig. 11). When the area which is in contact with the placement surface g is increased, stable placement can be realized also during use of the defibrillator 200 (Fig. 11).

While the present invention has been described with reference to certain embodiments thereof, the scope of the present invention is not limited to the embodiments described above, and it will be understood by those skilled in the art that various changes and modifications may be made therein without departing from the scope of the present invention as defined by the appended claims.

As described above, the medical equipment case 100 can be applied not only to to the defibrillator 200, but also to various kinds of medical equipment having a display (e.g., a transportation patient monitor), and the use of the medical equipment case should not be construed in a limiting sense.

## Claims

1. A medical equipment case (100) configured to cover at least a portion of a housing of a medical equipment (200), the medical equipment case (100) comprising:
a case body configured to cover at least a portion of the housing, the case body comprising a contact surface (124) and a bottom surface configured for placing the medical equipment (200) in a vertical manner on a placement surface (g);
a support member (120) comprising a contact surface (123) and a connecting portion (121) for connecting the support member (120) to the case body, wherein a portion of the support member (120) is connected to the case body, and another portion of the support member is configured to be attachable to and detachable from the case body;
**characterized in that** the support member (120) can be configured in a state wherein the support member (120) is closely attached to the case body and in an opened state wherein only the connecting portion (121) of the support member (120) is connected to the case body and wherein the contact surface (124) of the case body and the contact surface (123) of the support member (120) can be contacted with the placement surface (g) such that the support member (120) supports the load of the medical equipment (200) from a side opposite to a side on which a display (210) of the medical equipment (200) is disposed such that the display (210) of the medical equipment (200) is inclined with respect to the placement surface (g).

2. The medical equipment case (100) according to claim 1, wherein, in a state in which a contact surface (123) of the support member (120) is in contact with the case body, a surface on the side opposite to the side on which the display (210) of the medical equipment (200) is disposed has a substantially planar shape.

3. The medical equipment case (100) according to claim 1 or 2, further comprising fasteners (125, 126, 127, 128) provided on a contact surface (124) of the case body and the contact surface (123) of the support member (120) respectively.

4. The medical equipment case (100) according to claim 3, wherein the fasteners (125, 126, 127, 128) are magnets.

5. The medical equipment case (100) according to any one of claims 1 to 4, further comprising a grasping portion (122, 129) adapted to be grasped to pull the contact surface (124) of the case body and the contact surface (123) of the support member (120) apart from each other.

6. The medical equipment case (100) according to claim 5, wherein the grasping portion (122) is configured as a cutout portion into which fingers or palm of a user are insertable.

7. The medical equipment case (100) according to any one of claims 1 to 6, wherein each of the contact surface (123) of the support member (120) and the contact surface (124) of the case body has a substantially planar shape.

8. The medical equipment case (100) according to any one of claims 1 to 7, wherein the support member (120) is configured as a cushion member having elasticity.

9. The medical equipment case (100) according to any one of claims 1 to 8, wherein the support member (120) is provided at a lower portion of the medical equipment case (100) in a state in which the medical equipment case (100) is placed such that a bottom surface of the medical equipment case (100) is in contact with the placement surface (g).

10. The medical equipment case (100) according to any one of claims 1 to 7, wherein further comprising an angle adjusting mechanism via which the portion of the support member (120) and the case body are connected to each other such that a connection angle of the support member (120) with respect to the case body is adjustable.

11. The medical equipment case (100) according to claim 1, wherein the support member (120) is configured as a convex portion (120) which is convex in a direction away from a display (210) of the medical equipment (200).

## Patentansprüche

1. Koffer (100) medizinischer Ausrüstung, konfiguriert, um mindestens einen Abschnitt eines Gehäuses einer medizinischen Ausrüstung (200) zu bedecken, wobei der Koffer (100) medizinischer Ausrüstung umfasst:
einen Kofferkörper, konfiguriert, um mindestens einen Abschnitt des Gehäuses zu bedecken, wobei der Kofferkörper eine Kontaktoberfläche (124) und eine Bodenoberfläche umfasst, konfiguriert zum Platzieren der medizinischen Ausrüstung (200) auf eine vertikale Weise auf eine Platzierungsoberfläche (g);
ein Stützelement (120), umfassend eine Kontaktoberfläche (123) und einen Verbindungsabschnitt (121) zum Verbinden des Stützelements (120) mit dem Kofferkörper, wobei ein Abschnitt des Stützelements (120) mit dem Kofferkörper verbunden ist und ein anderer Abschnitt des Stützelements konfiguriert ist, um an dem Kofferkörper befestigbar und von diesem ablösbar zu sein;
**dadurch gekennzeichnet, dass** das Stützelement (120) in einem Zustand konfiguriert werden kann, in dem das Stützelement (120) eng an dem Kofferkörper befestigt ist, und in einem geöffneten Zustand, wobei ausschließlich der Verbindungsabschnitt (121) des Stützelements (120) mit dem Kofferkörper verbunden ist und wobei die Kontaktoberfläche (124) des Kofferkörpers und die Kontaktoberfläche (123) des Stützelements (120) mit der Platzierungsoberfläche (g) so in Kontakt gebracht werden kann, dass das Stützelement (120) die Last der medizinischen Ausrüstung (200) von einer Seite stützt, die einer Seite gegenüberliegt, auf der eine Anzeige (210) der medizinischen Ausrüstung (200) so angeordnet ist, dass die Anzeige (210) der medizinischen Ausrüstung (200) bezüglich der Platzierungsoberfläche (g) geneigt ist.

2. Koffer medizinischer Ausrüstung (100) nach Anspruch 1, wobei in einem Zustand, in dem eine Kontaktoberfläche (123) des Stützelements (120) mit dem Kofferkörper in Kontakt ist, eine Oberfläche auf der Seite, die der Seite gegenüberliegt, auf der die Anzeige (210) der medizinischen Ausrüstung (200) angeordnet ist, eine im Wesentlichen planare Form aufweist.

3. Koffer medizinischer Ausrüstung (100) nach Anspruch 1 oder 2, weiter umfassend Befestigungseinheiten (125, 126, 127, 128), jeweils auf einer Kontaktoberfläche (124) des Kofferkörpers und der Kontaktoberfläche (123) des Stützelements (120) bereitgestellt.

4. Koffer medizinischer Ausrüstung (100) nach Anspruch 3, wobei die Befestigungseinheiten (125, 126, 127, 128) Magnete sind.

5. Koffer medizinischer Ausrüstung (100) nach einem der Ansprüche 1 bis 4, weiter umfassend einen Greifabschnitt (122, 129), angepasst, um gegriffen au werden, um die Kontaktoberfläche (124) des Kofferkörpers und die Kontaktoberfläche (123) des Stützelements (120) voneinander wegzuziehen.

6. Koffer medizinischer Ausrüstung (100) nach Anspruch 5, wobei der Greifabschnitt (122) als ein Ausschnittsabschnitt konfiguriert ist, in den Finger oder Handfläche eines Anwenders einführbar sind.

7. Koffer medizinischer Ausrüstung (100) nach einem der Ansprüche 1 bis 6, wobei jede von der Kontaktoberfläche (123) des Stützelements (120) und der Kontaktoberfläche (124) des Kofferkörpers eine im Wesentlichen planare Form aufweist.

8. Koffer medizinischer Ausrüstung (100) nach einem der Ansprüche 1 bis 7, wobei das Stützelement (120) als ein Polsterelement, das Elastizität aufweist, konfiguriert ist.

9. Koffer medizinischer Ausrüstung (100) nach einem der Ansprüche 1 bis 8, wobei das Stützelement (120) an einem niedrigeren Abschnitt des Koffers medizinischer Ausrüstung (100) in einem Zustand bereitgestellt ist, in dem der Koffer medizinischer Ausrüstung (100) so platziert ist, dass eine Bodenoberfläche des Koffers medizinischer Ausrüstung (100) mit der Platzierungsoberfläche (g) in Kontakt ist.

10. Koffer medizinischer Ausrüstung (100) nach einem der Ansprüche 1 bis 7, wobei weiter umfassend einen Winkeleinstellungsmechanismus, mittels dem der Abschnitt des Stützelements (120) und des Kofferkörpers miteinander so verbunden werden, dass ein Verbindungswinkel des Stützelements (120) bezüglich des Kofferkörpers einstellbar ist.

11. Koffer medizinischer Ausrüstung (100) nach Anspruch 1, wobei das Stützelement (120) als ein konvexer Abschnitt (120) konfiguriert ist, der in einer Richtung weg von einer Anzeige (210) der medizinischen Ausrüstung (200) konvex ist.

## Revendications

1. Caisson d'équipement médical (100) configuré pour recouvrir au moins une partie d'un boîtier d'un équipement médical (200), le caisson d'équipement médical (100) comprenant:
un corps de caisson configuré pour recouvrir au moins une partie du boîtier, le corps de caisson comprenant une surface de contact (124) et une surface inférieure configurée pour placer l'équipement médical (200) de manière verticale sur une surface de placement (g) ;
un élément de support (120) comprenant une surface de contact (123) et une partie de connexion (121) pour connecter l'élément de support (120) au corps de caisson, dans lequel une partie de l'élément de support (120) est connectée au corps de caisson, et une autre partie de l'élément de support est configurée pour pouvoir être fixée au corps de caisson et pouvoir être séparée de celui-ci ;
**caractérisé en ce que** l'élément de support (120) peut être configuré dans un état dans lequel l'élément de support (120) est étroitement fixé au corps de caisson et dans un état ouvert dans lequel seule la partie de connexion (121) de l'élément de support (120) est connectée au corps de caisson et dans lequel la surface de contact (124) du corps de caisson et la surface de contact (123) de l'élément de support (120) peuvent être mises en contact avec la surface de placement (g) de telle sorte que l'élément de support (120) supporte la charge de l'équipement médical (200) depuis un côté opposé à un côté sur lequel un affichage (210) de l'équipement médical (200) est disposé de telle sorte que l'affichage (210) de l'équipement médical (200) est incliné par rapport à la surface de placement (g).

2. Caisson d'équipement médical (100) selon la revendication 1, dans lequel, dans un état dans lequel une surface de contact (123) de l'élément de support (120) est en contact avec le corps de caisson, une surface sur le côté opposé au côté sur lequel est disposé l'affichage (210) de l'équipement médical (200) présente une forme sensiblement plane.

3. Caisson d'équipement médical (100) selon la revendication 1 ou 2, comprenant en outre des fixations (125, 126, 127, 128) prévues respectivement sur une surface de contact (124) du corps de caisson et la surface de contact (123) de l'élément de support (120).

4. Caisson d'équipement médical (100) selon la revendication 3, dans lequel les fixations (125, 126, 127, 128) sont des aimants.

5. Caisson d'équipement médical (100) selon l'une quelconque des revendications 1 à 4, comprenant en outre une partie de saisie (122, 129) adaptée pour être saisie pour tirer la surface de contact (124) du corps de caisson et la surface de contact (123) de l'élément de support (120) l'une à l'écart de l'autre.

6. Caisson d'équipement médical (100) selon la revendication 5, dans lequel la partie de saisie (122) est configurée sous forme d'une partie découpée dans laquelle des doigts ou une paume d'un utilisateur peuvent être insérés.

7. Caisson d'équipement médical (100) selon l'une quelconque des revendications 1 à 6, dans lequel chacune de la surface de contact (123) de l'élément de support (120) et de la surface de contact (124) du corps de caisson présente une forme sensiblement plane.

8. Caisson d'équipement médical (100) selon l'une quelconque des revendications 1 à 7, dans lequel l'élément de support (120) est configuré sous forme d'un élément de coussin présentant une élasticité.

9. Caisson d'équipement médical (100) selon l'une quelconque des revendications 1 à 8, dans lequel l'élément de support (120) est prévu au niveau d'une partie inférieure du caisson d'équipement médical (100) dans un état dans lequel le caisson d'équipement médical (100) est placé de telle sorte qu'une surface inférieure du caisson d'équipement médical (100) est en contact avec la surface de placement (g).

10. Caisson d'équipement médical (100) selon l'une quelconque des revendications 1 à 7, comprenant en outre un mécanisme d'ajustement d'angle via lequel la partie de l'élément de support (120) et le corps de caisson sont connectés l'un à l'autre de telle sorte qu'un angle de connexion de l'élément de support (120) par rapport au corps de caisson est ajustable.

11. Caisson d'équipement médical (100) selon la revendication 1, dans lequel l'élément de support (120) est configuré sous forme d'une partie convexe (120) qui est convexe dans une direction s'éloignant d'un affichage (210) de l'équipement médical (200).
